(19) Europäisches Patentamt

European Patent Office

Office européen des brevets

(11) Veröffentlichungsnummer : **0 200 030**
**B1**

(12) **EUROPÄISCHE PATENTSCHRIFT**

(45) Veröffentlichungstag der Patentschrift :
15.06.88

(21) Anmeldenummer : 86104676.1

(22) Anmeldetag : 05.04.86

(51) Int. Cl.⁴ : **C 07 C 69/54**, C 07 C 67/08,
C 07 C 67/48, C 08 F 220/20

(54) **Verfahren zur Herstellung von (Meth)Acrylsäureestern und deren Verwendung.**

Ein Antrag gemäss Regel 88 EPÜ auf Berichtigung der Beschreibung liegt vor. Über diesen Antrag wird im Laufe des Verfahrens von der Prüfungsabteilung eine Entscheidung getroffen werden.

(30) Priorität : 20.04.85 DE 3514402

(43) Veröffentlichungstag der Anmeldung :
05.11.86 Patentblatt 86/45

(45) Bekanntmachung des Hinweises auf die Patenterteilung : 15.06.88 Patentblatt 88/24

(84) Benannte Vertragsstaaten :
AT BE DE FR GB IT NL SE

(56) Entgegenhaltungen :
FR-A- 1 030 459
FR-A- 2 376 117

(73) Patentinhaber : BAYER AG
Konzernverwaltung RP Patentabteilung
D-5090 Leverkusen 1 Bayerwerk (DE)

(72) Erfinder : Meixner, Jürgen, Dr.
Bethelstrasse 18
D-4150 Krefeld 1 (DE)
Erfinder : Pedain, Josef, Dr.
Haferkamp 6
D-5000 Köln 80 (DE)
Erfinder : Höhlein, Peter, Dr.
Windmühlenweg 3 E
D-4152 Kempen 3 (DE)
Erfinder : Margotte, Dieter, Dr.
Wedelstrasse 48
D-4150 Krefeld 1 (DE)

EP 0 200 030 B1

**Beschreibung**

Die Erfindung betrifft ein ·Verfahren zur Herstellung von (Meth)Acrylsäureestern mit geringer Säurezahl durch Verestern von (Meth)Acrylsäure mit ein- bzw. mehrwertigen Alkoholen und anschließender Behandlung mit Carbodiimiden und deren Verwendung als Reaktivverdünner und als Co-Monomere.

Der Einsatz von (Meth)Acrylsäureestern von ein- bzw. mehrwertigen Alkoholen als Reaktivverdünner oder Co-Monomere, bevorzugt als Reaktivverdünner in strahlenhärtbaren Überzugsmassen, ist grundsätzlich bekannt. Die Herstellung solcher (Meth)Acrylsäureester erfolgt im allgemeinen durch azeotrope Veresterung von (Meth)Acrylsäure mit den entsprechenden Alkoholen in einem inerten Lösungsmittel unter Verwendung eines sauren Veresterungskatalysators in Gegenwart von Stabilisatoren. Nach beendeter Veresterung wird der Katalysator ausgewaschen und das Lösungsmittel abdestilliert.

Auch wenn im Zusammenhang mit dem Auswaschen des Katalysators eine Neutralisation vorgenommen wird, bleibt meist eine Restsäurezahl von bis zu 3 (mg KOH/g Substanz) bestehen. Die Beibehaltung der Restsäurezahl bedeutet jedoch, daß sich die Reste von (Meth)Acrylsäure durch Geruch, durch die Korrosion von Behältern und evtl. Hautreizungen bei der Handhabung bemerkbar machen.

Man hat versucht, das Problem dadurch zu lösen, daß nach der Herstellung des (Meth)Acrylsäureesters gelöschter Kalk zugegeben und das entstehende unlösliche Calciumsalz durch Filtration entfernt wird (US 3.717.672). Hierbei bilden sich jedoch oft schmierige und nur schwer filtrierbare Niederschläge.

Weiterhin ist ein Verfahren zur Herstellung von (Meth)Acrylsäureestern bekannt, bei dem nach der Neutralisation des Veresterungskatalysators die restliche (Meth)Acrylsäure mit einer Epoxidverbindung umgesetzt wird (EP 127 766). Auch hierbei entstehen jedoch Produkte mit Säurezahlen, die größer als 1 (mg KOH/g Substanz) sind. Je nach Art und Menge des eingesetzten Epoxids ändert sich dabei zusätzlich die Viskosität des Endproduktes in unterschiedlichem Ausmaß.

Es war daher wünschenswert, (Meth)Acrylsäureester so herzustellen, daß das Endprodukt eine noch weiter verringerte Säurezahl besitzt, ohne daß die Eigenschaften der damit hergestellten polymerisationsfähigen Gemische, wie deren Farbzahl, Viskosität oder Reaktivität, nachteilig beeinflußt werden; gleichzeitig sollte die Reaktionszeit zur Herstellung der (Meth)Acrylsäureester nicht zu sehr verlängert werden.

Die Aufgabe wurde dadurch gelöst, daß die restliche (Meth)Acrylsäure nach der Umsetzung der Veresterungskomponenten mit einem Carbodiimid umgesetzt wird. Hierbei wird als verringerte Säurezahl die deutliche Verbesserung auf Werte unter 0,5 (mg KOH/g Substanz), vielfach auf höchstens 0,3 erzielt.

Die Erfindung betrifft demnach ein Verfahren zur Herstellung von Estern der (Meth)Acrylsäure durch säurekatalysierte Umsetzung von (Meth)Acrylsäure mit Alkoholen in einem mit Wasser nicht mischbaren Lösungsmittel, das dadurch gekennzeichnet ist, daß man das Reaktionsgemisch zunächst mit wäßrigen, alkalisch reagierenden Lösungen wäscht, mit Wasser neutral wäscht und dann bei erhöhter Temperatur mit Carbodiimiden umsetzt.

Als Säure im erfindungsgemäßen Verfahren kann sowohl Acrylsäure als auch Methacrylsäure sowie ein Gemisch beider eingesetzt werden.

Als Alkohole für das erfindungsgemäße Verfahren können ein- oder mehrwertige, gesättigte, aliphatische oder cycloaliphatische Alkohole eingesetzt werden, die gegebenenfalls Ethergruppen enthalten. Solche Alkohole haben Molekulargewichte von 32 bis etwa 400. Beispielhaft seien folgende Alkohole genannt: Methanol, Ethanol, Propanole, Butanole, Hexanole, Cetylalkohol, Stearylalkohol, Ethylenglykol, Propylenglykol, Butandiol-1,4, Pentadiol-1,5, Neopentylglykol, Hexandiol-1,6, Glycerin, Trimethylolethan, Trimethylolpropan, Pentaerythrit, 2-Ethylhexanol, Cyclohexanol, Dimethylolcyclohexan sowie Oxalkylierungsprodukte der genannten Alkohole mit beispielsweise 1-5 Mol Ethylenoxid oder Propylenoxid pro Hydroxyl-Äquivalent. Für den Fall der Verwendung von erfindungsgemäß hergestellten (Meth)Acrylsäureestern als Co-Monomere wird bevorzugt von niederen Alkoholen, beispielsweise solchen mit 1-6 C-Atomen, bevorzugt 1-2 C-Atomen ausgegangen, während man beim Einsatz der (Meth)Acrylsäureester als Reaktivverdünner von höheren Alkoholen, beispielsweise solchen mit Molekulargewichten von 62 bis etwa 400, ausgeht.

Im allgemeinen werden 70-150 Mol-% Acryl- und/oder Methacrylsäure, bevorzugt 85-120 Mol-%, besonders bevorzugt 95-110 Mol-%, bezogen auf vorhandene Hydroxyl-Äquivalente, eingesetzt.

Als saure Veresterungskatalysatoren können anorganische oder organische Säuren oder saure Ionenaustauscher in einer Menge von 0,1-3 Gew.-%, bezogen auf das Gewicht der zu veresternden Reaktionskomponenten, eingesetzt werden. Beispiele für solche Veresterungskatalysatoren sind Schwefelsäure, Phosphorsäure, Pyrophosphorsäure, p-Toluolsulfonsäure, Styrol-divinylbenzol-sulfonsäure-Kationenaustauscher, Chorsulfonsäure, Chlorameisensäure, bevorzugt Schwefelsäure und p-Toluolsulfonsäure.

Das erfindungsgemäße Verfahren wird in einem Lösungsmittel durchgeführt, das mit Wasser nicht mischbar ist und mit Wasser im Sinne einer Wasserdampf-Destillation destillierbar ist. Hierfür kommen Kohlenwasserstoffe sowie deren Halogen- oder Nitro-Substitutionsprodukte in Betracht sowie weitere Lösungsmittel, die weder mit den Reaktionspartnern reagieren noch sich unter dem Einfluß der sauren Katalysatoren verändern. In bevorzugter Weise werden nicht substituierte Kohlenwasserstoffe eingesetzt. Beispielhaft seien genannt: aliphatische Kohlenwasserstoffe, wie Hexan, Heptan, Octan, Benzinfraktionen verschiedener Siedebereiche, cycloaliphatische Kohlenwasserstoffe, wie Cyclopentan, Cyclohexan, Methyl-cyclohexan, oder aromatische Kohlenwasserstoffe, wie Benzol, Toluol oder die isomeren Xylole. In bevorzugter Weise werden solche Lösungsmittel eingesetzt, die im Bereich von 70-120 °C sieden. Insbesondere seien hier Cyclohexan, Toluol oder Benzinfraktionen im Siedebereich von 70-120 °C genannt. Das mit Wasser nicht mischbare Lösungsmittel kann auch ein Gemisch der obengenannten Stoffe sein. Es wird in einer Menge von 10-100 Gew.-%, bevorzugt 15-50 Gew.-%, besonders bevorzugt 20-40 Gew.-%, bezogen auf das Gewicht der zu veresternden Reaktionskomponente, eingesetzt.

Das erfindungsgemäße Verfahren kann in Gegenwart eines oder mehrerer Polymerisationsinhibitoren in einer Menge von 0,01-1 Gew.-%, bevorzugt 0,1-0,5 Gew.-%, bezogen auf das zu veresternde Gemisch aus (Meth)Acrylsäure und Alkohol, durchgeführt werden. Solche Inhibitoren sind beispielsweise in Houben-Weyl, Methoden der organischen Chemie, 4. Auflage, Band XIV/1, Georg Thieme Verlag, Stuttgart 1961, Seite 433 ff. beschrieben. Als Beispiele seien genannt : Natriumdithionit, Natriumhydrogensulfid, Schwefel, Hydrazin, Phenylhydrazin, Hydrazobenzol, N-Phenyl-β-naphthylamin, N-Phenyl-ethanolamin, Dinitro-benzol, Picrinsäure, p-Nitroso-dimethylanilin, Diphenylnitrosamin, Phenole, wie p-tert.-Butyl-brenzcatechin, 2,5-Di-tert.-amyl-hydrochinon, p-Alkoxyphenole, Di-tert.-butylhydrochinon, Tetramethyl-thiuramdisulfid, 2-Mercaptobenzthiazol und Dimethyl-dithiocarbaminsäure-natriumsalz.

Weiterhin wird in einer bevorzugten Variante des erfindungsgemäßen Verfahrens ein sauerstoffhaltiges Gas, vorzugsweise Luft, in das Reaktionsgemisch eingeleitet.

Das erfindungsgemäße Verfahren wird zunächst zur Veresterung in einem Temperaturbereich von 60-140 °C, bevorzugt 70-120 °C, besonders bevorzugt beim Siedepunkt des eingesetzten Lösungsmittels durchgeführt. Hierbei wird ständig Lösungsmittel aus dem Reaktionsgemisch destillativ abgezogen, außerhalb des Reaktionsgefäßes in einem Wasserabscheider von herausgeschleppten Wasser abgetrennt und danach wieder in das Reaktionsgemisch zurückgeführt. Das Ende der Reaktion ist erreicht, wenn kein weiteres Reaktionswasser mehr aus dem Reaktionsgefäß herausgeschleppt wird.

Nach beendeter Veresterung wird das Reaktionsgemisch mit einer wäßrigen alkalischen Lösung, wie verdünnte Natronlauge, verdünnte Kalilauge oder einer wäßrigen Lösung von Alkalicarbonaten, Alkalihydrogencarbonaten oder Ammoniak, bevorzugt mit verdünnter wäßriger Natronlauge, gewaschen. Danach wird das Reaktionsgemisch mit Wasser neutral gewaschen.

Danach wird das Reaktionsgemisch mit einem oder mehreren Carbodiimid(en) bei einer Temperatur von 40-120 °C, bevorzugt 50-100 °C, besonders bevorzugt 70-90 °C, so lange umgesetzt, bid die Säurezahl auf den gewünschten Wert gefallen ist, beispielsweise auf einen Wert von < 0,5 (mg KOH/g Substanz). Zum Umsatz mit Carbodiimid bestimmt man den nach der oben beschriebenen Wäsche erhaltenen Säuregrad und setzt dann Carbodiimid(e) im Verhältnis von 1-4 Mol pro Äquivalent Carboxylgruppe zu ; bevorzugt ist ein Molverhältnis von 1,0-2,5 Mol Carbodiimid pro Carboxyl-Äquivalent.

Beispiele für erfindungsgemäß einsetzbare Carbodiimide sind solche mit aliphatischen, cycloaliphatischen, aromatischen oder aromatisch-aliphatischen Substituenten, wie Diethylcarbodiimid, Dicyclohexylcarbodiimid, Diphenylcarbodiimid, Dibenzylcarbodiimid, Di-(isopropylphenyl)-carbodiimid, Di-(methylphenyl)-carbodiimid sowie Polycarbodiimide aus der Reaktion von Carbodiimiden mit Diisocyanaten. Carbodiimide und Polycarbodiimide der genannten Art sind beispielsweise bekannt aus Angew. Chem. 74, 1962, S. 801, besonders S. 806.

Die Umsetzung des gewaschenen Veresterungsgemisches mit Carbodiimid kann in Gegenwart oder bei Abwesenheit des mit Wasser nicht mischbaren Lösungsmittels durchgeführt werden. In bevorzugter Weise wird die Umsetzung mit dem Carbodiimid durchgeführt, nachdem das mit Wasser nicht mischbare Lösungsmittel vom neutral gewaschenen Reaktionsgemisch abdestilliert wurde, beispielsweise unter vermindertem Druck, etwa 10-50 mbar, und erhöhter Temperatur, beispielsweise 70-100 °C.

Wegen der zu erwartenden unübersichtlichen Reaktionen zwischen dem Carbodiimid und dem (Meth)Acrylsäureester, wobei unerwünschte Änderungen der Viskosität, der Farbzahl und der Reaktivität erwartet werden konnten, ist die glatte und erfolgreiche Durchführung des erfindungsgemäßen Verfahrens überraschend.

Die erfindungsgemäß erhältlichen (Meth)Acrylsäureester finden Verwendung als Reaktivverdünner oder als Co-Monomere in Mischungen aus anderen olefinisch ungesättigten Verbindungen. Insbesondere finden sie Verwendung als Reaktivverdünner in strahlenhärtbaren Überzugsmassen. Die Erfindung betrifft daher auch diese Verwendungen der im beschriebenen Verfahren erhältlichen (Meth)Acrylsäureester.

## Beispiele

In den nachfolgenden Beispielen beziehen sich die Prozentangaben auf das Gewicht der zu veresternden Reaktionskomponenten. Die Viskositätsmessungen wurden im Rheometer der Firma Contraves bei 20 °C durchgeführt. Die Jodfarbzahlen wurden nach DIN 6162 bestimmt.

Bei den Beispielen wurde die Kondensation 70 %ig in Toluol in Gegenwart von 0,75 % p-Toluolsulfonsäure als Katalysator und 0,1 % p-Methoxyphenol und 0,1 % Di-tert.-butylhydrochinon als Inhibitoren durchgeführt.

Die in der Tabelle aufgeführten Ausgangskomponenten sowie Katalysator und Inhibitoren wurden in Toluol unter Luftzufuhr so lange bei 110-120 °C erhitzt, bis sich kein Wasser mehr abschied. Nach Abkühlen auf 20-30 °C wurde die organische Phase einmal mit verdünnter wäßriger Natronlauge und zweimal mit Wasser gewaschen. Daraufhin wurde bei 80-90 °C und 50-60 mbar das Toluol abdestilliert. Nach Messung der Säurezahl wurde das jeweilige Carbodiimid (siehe Tabelle) zugegeben und die Mischung bei 80 °C so lange gehalten, bis die Säurezahl den gewünschten Wert erreicht hatte (ca. 1 bis 4 Stunden). Anschließend wurde filtriert, und es wurden die Endkennzahlen gemessen, die in der Tabelle aufgeführt sind.

Tabelle

| Ausgangskomponenten (Teile) | Beispiele | | |
|---|---|---|---|
| | 1 | 2 | 3 |
| Acrylsäure | 191 | 120 | 139 |
| Hexandiol-1,6 | 156 | - | - |
| Tripropylenglykol | - | 169 | 195 |
| **Daten nach Kondensation** | | | |
| Säurezahl (mg KOH/g Substanz) | 5,0 | 2,4 | 2,4 |
| Viskosität (mPa.s) | 8 | 16 | 16 |
| Farbzahl | 0-1 | 1-2 | 1-2 |
| **Zugabe von (Teile)** | | | |
| Dicyclohexylcarbodiimid | 5,5 | 2,3 | - |
| Diisopropylphenylcarbodiimid | - | - | 7,1 |
| **Enddaten** | | | |
| Säurezahl (mg KOH/g Substanz) | 0,4 | 0,2 | 0,2 |
| Viskosität (mPa.s) | 8 | 16 | 16 |
| Farbzahl | 0-1 | 1-2 | 1-2 |

**Patentansprüche**

1. Verfahren zur Herstellung von Estern der (Meth)Acrylsäure durch säurekatalysierte Umsetzung von (Meth)Acrylsäure mit Alkoholen in einem mit Wasser nicht mischbaren Lösungsmittel, dadurch gekennzeichnet, daß man das Reaktionsgemisch zunächst mit wäßrigen, alkalisch reagierenden Lösungen wäscht, mit Wasser neutral wäscht und dann bei erhöhter Temperatur mit Carbodiimiden umsetzt.

2. Verfahren nach Anspruch 1, dadurch gekennzeichnet, daß 1-4 Mol Carbodiimid pro Äquivalent Restsäure eingesetzt werden.

3. Verfahren nach Ansprüchen 1 und 2, dadurch gekennzeichnet, daß 1,0-2,5 Mol Carbodiimid pro Äquivalent Restsäure eingesetzt werden.

4. Verfahren nach Ansprüchen 1 bis 3, dadurch gekennzeichnet, daß die Umsetzung mit Carbodiimiden bei 40-120 °C durchgeführt wird.

5. Verfahren nach Ansprüchen 1 bis 4, dadurch gekennzeichnet, daß die Umsetzung mit Carbodiimiden bei 50-100 °C durchgeführt wird.

6. Verfahren nach Ansprüchen 1 bis 5, dadurch gekennzeichnet, daß das mit Carbodiimid umzusetzende Reaktionsgemisch unter Durchleiten eines sauerstoffhaltigen Gases hergestellt wurde.

7. Verfahren nach Ansprüchen 1 bis 6, dadurch gekennzeichnet, daß vor der Umsetzung mit Carbodiimiden das mit Wasser nicht mischbare Lösungsmittel vom neutral gewaschenen Reaktionsgemisch abdestilliert wird.

8. Verwendung der nach Ansprüchen 1 bis 7 hergestellten (Meth)Acrylsäureester als Reaktivverdünner oder Co-Monomere.

**0 200 030**

9. Verwendung der (Meth)Acrylsäureester nach Anspruch 8, dadurch gekennzeichnet, daß die (Meth)Acrylsäureester als Reaktivverdünner in strahlenhärtbaren Überzugsmassen eingesetzt werden.

**Claims**

1. A process for the production of esters of (meth)-acrylic acid by acid-catalyzed reaction of (meth)acrylic acid with alcohols in a water-immiscible solvent, characterized in that the reaction mixture is first washed with aqueous, alkaline-reacting solutions and is then washed with water until neutral and then reacted with carbodiimides at elevated temperature.

2. A process as claimed in Claim 1, characterized in that 1 to 4 moles carbodiimide are used per equivalent residual acid.

3. A process as claimed in Claims 1 and 2, characterized in that 1.0 to 2.5 moles carbodiimide are used per equivalent residual acid.

4. A process as claimed in Claims 1 to 3, characterized in that the reaction with carbodiimides is carried out at 40 to 120 °C.

5. A process as claimed in Claims 1 to 4, characterized in that the reaction with carbodiimides is carried out at 50 to 100 °C.

6. A process as claimed in Claims 1 to 5, characterized in that an oxygen-containing gas is passed through the reaction mixture to be reacted with carbodiimide during its preparation.

7. A process as claimed in Claims 1 to 6, characterized in that the water-immiscible solvent is distilled off from the reaction mixture washed until neutral before the reaction with carbodiimides.

8. The use of the (meth)acrylic acid esters produced by the process claimed in Claims 1 to 7 as reactive diluents or co-monomers.

9. The use of the (meth)acrylic acid esters as claimed in Claim 8, characterized in that the (meth)acrylic acid esters are used as reactive diluents in radiation-hardenable coating compositions.

**Revendications**

1. Procédé de production d'esters d'acide-(méth)acrylique par réaction, catalysée par un acide, d'acide (méth)acrylique avec des alcools dans un solvant non miscible à l'eau, caractérisé en ce qu'on lave le mélange réactionnel tout d'abord avec des solutions aqueuses à réaction alcaline, on le lave à l'eau jusqu'à neutralité puis on le fait réagir à température élevée avec des carbodiimides.

2. Procédé suivant la revendication 1, caractérisé en ce qu'on utilise 1 à 4 moles de carbodiimide par équivalent d'acide résiduel.

3. Procédé suivant les revendications 1 et 2, caractérisé en ce qu'on utilise 1,0 à 2,5 moles de carbodiimide par équivalent d'acide résiduel.

4. Procédé suivant les revendications 1 à 3, caractérisé en ce qu'on conduit la réaction avec des carbodiimides à 40-120 °C.

5. Procédé suivant les revendications 1 à 4, caractérisé en ce qu'on conduit la réaction avec des carbodiimides à 50-100 °C.

6. Procédé suivant les revendications 1 à 5, caractérisé en ce que le mélange réactionnel devant réagir avec le carbodiimide a été préparé par passage d'un courant de gaz contenant de l'oxygène.

7. Procédé suivant les revendications 1 à 6, caractérisé en ce que le solvant non miscible à l'eau est chassé par distillation du mélange réactionnel lavé jusqu'à neutralité avant la réaction avec des carbodiimides.

8. Utilisation des esters d'acide (méth)acrylique préparés suivant les revendications 1 à 7 comme diluants réactifs ou comme comonomères.

9. Utilisation des esters d'acide (méth)acrylique suivant la revendication 8, caractérisée en ce que les esters d'acide (méth)acrylique sont utilisés comme diluants réactifs dans des compositions de revêtement radiodurcissables.